Europäisches Patentamt

European Patent Office

Office européen des brevets

(19)

(11) Veröffentlichungsnummer : **0 014 989**
**B1**

(12) **EUROPÄISCHE PATENTSCHRIFT**

(45) Veröffentlichungstag der Patentschrift :
03.02.82

(51) Int. Cl.³ : **A 61 M 15/00**

(21) Anmeldenummer : **80100819.4**

(22) Anmeldetag : **19.02.80**

(54) **Inhalationsgerät.**

(30) Priorität : **24.02.79 DE 2907348**

(43) Veröffentlichungstag der Anmeldung :
**03.09.80 (Patentblatt 80/18)**

(45) Bekanntmachung des Hinweises auf die Patenterteilung : **03.02.82 Patentblatt 82/05**

(84) Benannte Vertragsstaaten :
**AT BE CH DE FR GB IT LU NL SE**

(56) Entgegenhaltungen :
**LU - A - 78 476**
**US - A - 3 561 444**

(73) Patentinhaber : **C.H. BOEHRINGER SOHN**
**Postfach 200**
**D-6507 Ingelheim am Rhein (DE)**

(72) Erfinder : **Zierenberg, Bernd, Dr.**
**Velt-Stoss-Strasse 4**
**D-6507 Ingelheim (DE)**

## Inhalationsgerät

Aus den DE-A-20 32 433, 23 08 584, 24 45 791 und 23 37 765 sowie der DE-C-26 37 162 sind Flüssigkeitszerstäuber bekannt, mit denen sich aus arzneimittelhaltigen Lösungen inhalierbare Aerosole herstellen lassen. Bei diesen Geräten werden die Schwingungen eines piezoelektrischen Schwingsystems auf eine Zerstäuberplatte übertragen. Die Zerstäuberplatte erzeugt aus der ihr zugeführten Flüssigkeit feine Tröpfchen, die mit der Luft ein Aerosol bilden.

Bei der Anwendung von Inhalationspräparaten für die Bronchospasmolyse ist es wesentlich, daß ein ausreichender Anteil der Tröpfchen des Aerosols klein genug ist, um mit der Atemluft an den Wirkungsort in der Lunge transportiert zu werden. Sind die Tröpfchen zu groß, schlagen sie sich bereits im Mund oder in den oberen Atemwegen nieder. Die angestrebte Wirkung wird dann nicht oder nicht in ausreichendem Maß erreicht. Ein Nachteil der bekannten Flüssigkeitszerstäuber ist, daß sie einen zu hohen Anteil relativ großer Tröpfchen liefern.

Es wurde nun gefunden, daß überraschenderweise die Beschichtung der Zerstäuberplatte in Geräten der oben genannten Art mit einem dünnen Elastomerenfilm eine wesentliche Verbesserung des Teilchenspektrums in dem erzeugten Aerosol ergibt.

Die Erfindung bezieht sich somit auf ein Inhalationsgerät mit einer Zerstäuberplatte, auf die die Schwingungen eines piezoelektrischen Schwingsystems übertragen werden, und ist dadurch gekennzeichnet, daß die Arbeitsfläche der Zerstäuberplatte mit einer dünnen Schicht eines Elastomeren überzogen ist.

Die Schichtdicke des Elastomeren kann zwischen 50 und 500 μm liegen, vorzugsweise beträgt sie zwischen 100 und 300 μm.

Die Zerstäuberplatte besteht vorzugsweise aus einem Metall mit geeigneten physikalischen und chemischen Eigenschaften, insbesondere Edelstahl. Für die Beschichtung eignen sich die verschiedensten Elastomeren. Sie werden z.B. in Form konzentrierter Polymerlösungen, aus denen das Lösungsmittel durch Verdunsten entfernt wird, oder durch Polymerisation von Monomeren bzw. Praepolymeren auf der Arbeitsfläche aufgebracht. Bei der Verwendung von Polyurethanen, die auf der Arbeitsfläche gebildet werden, ist — außer der Reinigung der Zerstäuberplatte — keine Vorbehandlung nötig. Werden andere Elastomere aufgebracht, kann die Vorbehandlung der Arbeitsfläche mit einem Primer die Bindung Elastomer/Metall verbessern.

Die Beschichtung einer Zerstäuberplatte aus Edelstahl (nach DE-A-2 308 584) ist im folgenden Beispiel beschrieben :

Beispiel

Die Arbeitsfläche der Zerstäuberplatte eines Inhalationsgeräts nach der DE-A-2 308 584 wird mit Methylenchlorid gereinigt. Auf der Arbeitsfläche werden 50 mg einer feuchtigkeitshärtenden Polyurethan-Vorstufe (50-prozentige Lösung von Di-isocyanatphenylmethan und einem mehrwertigen Polyätheralkohol in wasserfreiem Toluol, auf eine Viskosität von 23 Poise eingestellt) aufgebracht. Bei horizontaler Stellung der Zerstäuberplatte verteilt sich die Mischung derart über die Arbeitsfläche, daß eine gleichmäßige Schicht entsteht. Der Härtungsvorgang ist nach 24 Stunden abgeschlossen (ca. 18 °C, 50 % relative Luftfeuchtigkeit). Es resultiert ein Film von 200 μm Schichtdicke mit einer Bruchdehnung von 340 % und einer Zugfestigkeit von 3,5 N (0,35 kg).

Bei der Anwendung des auf diese Weise beschichteten Inhalationsgeräts wurde mit einer Dosis von 200 μg Fenoterol im Hundeversuch (Acetylcholin-Bronchospasmus) eine ebenso gute Wirkung erzielt wie mit einem handelsüblichen treibgasbetriebenen Dosier-aerosol und derselben Dosis pro Hub.

Bei der Verwendung des Geräts nach der DE-A-2 308 584 zeigte sich dagegen im Vergleichsversuch keine Wirkung.

**Ansprüche**

1. Inhalationsgerät mit einer Zerstäuberplatte, auf die die Schwingungen eines piezoelektrischen Schwingsystems übertragen werden, dadurch gekennzeichnet, daß die Arbeitsfläche der Zerstäuberplatte mit einer dünnen Schicht eines Elastomeren überzogen ist.

2. Inhalationsgerät nach Anspruch 1, dadurch gekennzeichnet, daß die Elastomerschicht auf der Arbeitsfläche eine Dicke von 50 bis 500 μm, vorzugsweise von 100 bis 300 μm hat.

3. Inhalationsgerät nach Anspruch 1 oder 2, dadurch gekennzeichnet, daß die Elastomerschicht aus einem Polyurethan besteht.

**Claims**

1. Inhaler having an atomiser device, known *per se,* based on a piezoelectric oscillatory system, characterised in that the working surface of the atomiser plate is coated with a thin layer of an elastomer.

2. Inhaler as claimed in claim 1, characterised in that the layer of elastomer on the working surface has a thickness of 50 to 500 μm, preferably 100 to 300 μm.

3. Inhaler as claimed in claim 1 or 2, characterised in that the layer of elastomer consists of a polyurethane.

**Revendications**

1. Appareil pour inhalations avec un dispositif pulvérisateur connu en soi, à base d'un système oscillant piézoélectrique, caractérisé en ce que la surface de travail de la plaque du pulvérisateur est revêtue d'une mince couche d'un élastomère.

2. Appareil pour inhalations selon la revendication 1, caractérisé en ce que la couche d'élastomère sur la surface de travail a une épaisseur de 50 à 500 μm, de préférence de 100 à 300 μm.

3. Appareil pour inhalations selon la revendication 1 ou 2, caractérisé en ce que la couche d'élastomère est constituée d'un polyuréthane.